# EUROPÄISCHE PATENTSCHRIFT

(11) **EP 1 448 530 B1**
(45) Veröffentlichungstag und Bekanntmachung des Hinweises auf die Patenterteilung: **03.10.2007**
(21) Anmeldenummer: 02781305.4
(22) Anmeldetag: 06.11.2002
(51) Int. Cl.: C07D 215/48, C07D 241/46, C07D 453/02, A61P 25/28, A61K 31/47, A61K 31/517

(54) **HETEROARYLCARBONSÄUREAMIDE**
HETEROARYL CARBOXYLIC ACID AMIDES
AMIDES D'ACIDE HETEROARYLCARBOXYLIQUE

(30) Priorität: 19.11.2001 DE 10156719
(43) Veröffentlichungstag der Anmeldung: 25.08.2004
(73) Patentinhaber: Bayer HealthCare AG, 51368 Leverkusen (DE)
(72) Erfinder: HENDRIX, Martin, 51519 Odenthal (DE); BÖSS, Frank-Gerhard, London Road, Berkshire SL5 7DF (GB); ERB, Christina, 65830 Kriftel (DE); KRÜGER, Joachim, 40474 Düsseldorf (DE); LUITHLE, Joachim, 42489 Wülfrath (DE); METHFESSEL, Christoph, 42327 Wuppertal (DE); SCHREIBER, Rudy, Menlo Park, CA 94025 (US); WIESE, Welf-Burkhard, 42929 Wermelskirchen (DE)
(86) Internationale Anmeldenummer: PCT/EP2002/012375
(87) Internationale Veröffentlichungsnummer: WO 2003/043991

(56) Entgegenhaltungen:
- EP-A- 0 327 335
- WO-A-01/60821
- WO-A-85/01048
- WO-A-91/17161
- DE-A- 3 724 059

## Beschreibung

Die Erfindung betrifft neue Heteroarylcarbonsäureamide, Verfahren zu ihrer Herstellung sowie ihre Verwendung zur Herstellung von Arzneimitteln zur Behandlung und/oder Prophylaxe von Krankheiten und zur Verbesserung der Wahrnehmung, Konzentrationsleistung, Lernleistung und/oder Gedächtnisleistung.

Nikotinische Acetylcholin-Rezeptoren (nAChR) bilden eine große Familie von Ionenkanälen, die durch den körpereigenen Botenstoff Acetylcholin aktiviert werden (Galzi and Changeux, Neuropharmacol. 1995, 34, 563-582). Ein funktioneller nAChR besteht aus fünf Untereinheiten, die unterschiedlich (bestimmte Kombinationen von α1-9 und β1-4,γ,δ,ε-Untereinheiten) oder identisch (α7-9) sein können. Dies führt zur Bildung einer Vielfalt von Subtypen, die eine unterschiedliche Verteilung in der Muskulatur, dem Nervensystem und anderen Organen zeigen (McGehee and Role, Annu. Rev. Physiol., 1995, 57, 521-546). Aktivierung von nAChR führt zum Einstrom von Kationen in die Zelle und zur Stimulation von Nerven- oder Muskelzellen. Selektive Aktivierung einzelner nAChR-Subtypen beschränkt diese Stimulation auf die Zelltypen, die den entsprechenden Subtyp besitzen und kann so unerwünschte Nebeneffekte wie z.B. die Stimulierung von nAChR in der Muskulatur vermeiden. Klinische Experimente mit Nikotin und Experimente in verschiedenen Tiermodellen weisen auf eine Rolle von zentralen nikotinischen Acetylcholin-Rezeptoren bei Lern- und Gedächtnisvorgängen hin (z.B. Rezvani and Levin, Biol. Psychiatry 2001, 49, 258-267). Nikotinische Acetylcholinrezeptoren des alpha7-Subtyps (α7-nAChR) haben eine besonders hohe Konzentration in für Lernen und Gedächtnis wichtigen Hirnregionen, wie dem Hippocampus und dem cerebralen Cortex (Séguéla et al., J. Neurosci. 1993, 13, 596-604). Der α7-nAChR besitzt eine besonders hohe Durchlässigkeit für Calcium-Ionen, erhöht glutamaterge Neurotransmission, beeinflusst das Wachstum von Neuriten und moduliert auf diese Weise die neuronale Plastizität (Broide and Leslie, Mol. Neurobiol. 1999, 20, 1-16).

Die WO 85/01048 und die DE-A-3724059 beschreiben bestimmte Heteroarylcarbonsäureamide als Serotonin-M- bzw. 5HT₃-Antagonisten zur Behandlung von Arrhythmien und Schmerz bzw. Psychosen und Störungen des Wachheitszustandes.

Die EP-A-0 327 335 offenbart bestimmte Heteroarylcarbonsäureamide mit gedächtnisverbessernder Wirkung.

Isochinolincarbonsäureamide mit 5HT₃-antagonistischer Wirkung zur Behandlung von ZNS-Erkrankungen sind aus der WO 91/17161 bekannt.

Die WO 01/60821 offenbart Biarylcarbonsäureamide mit Affinität zum α7-nAChR zur Behandlung von Lern- und Wahrnehmungsstörungen.

Bestimmte 2- und 3-Chinolincarbonsäureamide werden in Orjales et al. Drug Des. Discovery 2000, 16, 271-279 als Liganden am 5HT₃-Rezeptor beschrieben.

Die vorliegende Erfindung betrifft Verbindungen der allgemeinen Formel (I), 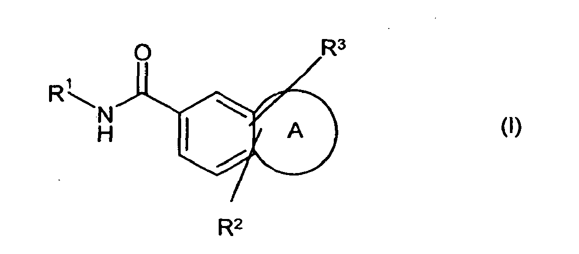 in welcher
- R¹: für I-Aza-bicyclo[2.2.2]Oct-3-yl steht, der Ring A für Pyrimido steht, oder für gegebenenfalls Benzo-kondensiertes Pyrido, Pyrazino oder Pyridazino steht,
und
- R² und R³: gleich oder verschieden sind und für Reste ausgewählt aus der Gruppe Wasserstoff, Halogen, Formyl, Carbamoyl, Cyano, Trifluormethyl, Trifluormethoxy, Nitro, (C₁-C₆)-Alkyl, (C₁-C₆)-Alkoxy, (C₁-C₆)-Alkylthio, (C₃-C₈)-Cycloalkyl, 4- bis 8-gliedriges Heterocyclyl, Phenyl oder 5- bis 6-gliedriges Heteroraryl stehen, wobei Phenyl und Heteroaryl gegebenenfalls durch Halogen, (C₁-C₄)-Alkyl, (C₁-C₄)-Alkoxy, Amino, Mono- oder Di-(C₁-C₄)-Alkylamino, (C₁-C₄)-Alkanoylamino oder (C₁-C₄)-Alkansulfonylamino substituiert sind.

In der allgemeinen Formel (I) können die Reste R² und R³ unabhängig voneinander an den Ring A oder an den Benzolring gebunden sein. Vorzugsweise sind die Reste R² und R³ an den Ring A gebunden.

Die erfindungsgemäßen Verbindungen können in stereoisomeren Formen, die sich entweder wie Bild und Spiegelbild (Enantiomere), oder die sich nicht wie Bild und Spiegelbild (Diastereomere) verhalten, existieren. Die Erfindung betrifft sowohl die Enantiomeren oder Diastereomeren oder deren jeweiligen Mischungen. Diese Mischungen der Enantiomere und Diastereomere lassen sich in bekannter Weise in die stereoisomer einheitlichen Bestandteile trennen.

Die erfindungsgemäßen Verbindungen können auch in Form ihrer Salze, Hydrate und/oder Solvate vorliegen.

Als Salze sind im Rahmen der Erfindung physiologisch unbedenkliche Salze der erfindungsgemäßen Verbindungen bevorzugt.

Physiologisch unbedenkliche Salze der erfindungsgemäßen Verbindungen können Säureadditionssalze der Verbindungen mit Mineralsäuren, Carbonsäuren oder Sulfonsäuren sein. Besonders bevorzugt sind z.B. Salze mit Chlorwasserstoffsäure, Bromwasserstoffsäure, Schwefelsäure, Phosphorsäure, Methansulfonsäure, Ehansulfonsäure, Toluolsulfonsäure, Benzolsulfonsäure, Naphthalindisulfonsäure, Essigsäure, Propionsäure, Milchsäure, Weinsäure, Zitronensäure, Fumarsäure, Maleinsäure oder Benzoesäure.

Als Salze können aber auch Salze mit üblichen Basen genannt werden, wie beispielsweise Alkalimetallsalze (z.B. Natrium- oder Kaliumsalze), Erdalkalisalze (z.B. Calcium- oder Magnesiumsalze) oder Ammoniumsalze, abgeleitet von Ammoniak oder organischen Aminen wie beispielsweise Diethylamin, Triethylamin, Ethyldiisopropylamin, Prokain, Dibenzylamin, N-Methylmorpholin, Dihydroabiethylamin, 1-Ephenamin oder Methyl-piperidin.

Hydrate der erfindungsgemäßen Verbindungen sind stöchiometrische Zusammensetzungen der Verbindungen oder seinen Salzen mit Wasser.

Solvate der erfindungsgemäßen Verbindungen sind stöchiometrische Zusammensetzungen der Verbindungen oder seinen Salzen mit Lösungsmittel.

Im Rahmen der vorliegenden Erfindung haben die Substituenten im Allgemeinen die folgende Bedeutung:
(C₁-C₄)-Alkanoylamino steht für einen geradkettigen oder verzweigten Alkanoylaminorest mit 1 bis 4 Kohlenstoffatomen. Bevorzugt ist ein geradkettiger oder verzweigter Alkanoylaminorest mit 1 bis 3 Kohlenstoffatomen. Beispielsweise und vorzugsweise seien genannt: Formylamino, Acetylamino, Propanoylamino, n-Butanoylamino, i-Butanoylamino.
(C₁-C₆)- und (C₁-C₄)-Alkoxy steht für einen geradkettigen oder verzweigten Alkoxyrest mit 1 bis 6 bzw. 1 bis 4 Kohlenstoffatomen. Bevorzugt ist ein geradkettiger oder verzweigter Alkoxyrest mit 1 bis 4, besonders bevorzugt mit 1 bis 3 Kohlenstoffatomen. Beispielsweise und vorzugsweise seien genannt: Methoxy, Ethoxy, n-Propoxy, Isopropoxy, tert.Butoxy, n-Pentoxy und n-Hexoxy.
(C₁-C₆)- und (C₁-C₄)-Alkyl stehen für einen geradkettigen oder verzweigten Alkylrest mit 1 bis 6 bzw. 1 bis 4 Kohlenstoffatomen. Bevorzugt ist ein geradkettiger oder verzweigter Alkylrest mit 1 bis 4, besonders bevorzugt mit 1 bis 3 Kohlenstoffatomen. Beispielsweise und vorzugsweise seien genannt: Methyl, Ethyl, n-Propyl, Isopropyl, tert.Butyl, n-Pentyl und n-Hexyl.
Mono-(C₁-C₄)-Alkylamino steht für einen geradkettigen oder verzweigten Alkylaminorest mit 1 bis 4 Kohlenstoffatomen. Bevorzugt ist ein geradkettiger oder verzweigter Alkylaminorest mit 1 bis 3 Kohlenstoffatomen. Beispielsweise und vorzugsweise seien genannt: Methylamino, Ethylamino, n-Propylamino, Isopropylamino, tert.Butylamino.
Di-(C₁-C₄)-alkylamino steht für einen geradkettigen oder verzweigten Dialkylaminorest, wobei die Alkylreste gleich oder verschieden sein können und jeweils 1 bis 4 Kohlenstoffatome enthalten. Bevorzugt ist ein geradkettiger oder verzweigter Dialkylaminorest, wobei der Alkylrest jeweils 1 bis 3 Kohlenstoffatome enthält. Beispielsweise und vorzugsweise seien genannt: Dimethylamino, Diethylamino, Di-n-propylamino, Diisopropylamino, Di-t-butylamino, Di-n-pentylamino, Di-n-hexylamino, Ethylmethylamino, Isopropylmethylamino, n-Butylethylamino.
(C₁-C₄)-Alkansulfonylamino stehen für einen geradkettigen oder verzweigten Alkansulfonylaminorest mit 1 bis 4 Kohlenstoffatomen. Bevorzugt ist ein geradkettiger oder verzweigter Alkansulfonylaminorest mit mit 1 bis 3 Kohlenstoffatomen. Beispielsweise und vorzugsweise seien genannt: Methansulfonylamino, Ethansulfonylamino, n-Propansulfonylamino, Isopropansulfonylamino, tert.Butansulfonylamino.
(C₁-C₆-Alkylthio steht für einen geradkettigen oder verzweigten Alkylthiorest mit 1 bis 6 Kohlenstoffatomen. Bevorzugt ist ein geradkettiger oder verzweigter Alkylthiorest mit 1 bis 4, besonders bevorzugt mit 1 bis 3 Kohlenstoffatomen. Beispielsweise und vorzugsweise seien genannt: Methylthio, Ethylthio, n-Propylthio, Isopropylthio, tert.Butylthio, n-Pentylthio und n-Hexylthio.
(C₃-C₈)-Cycloalkyl steht für Cyclopropyl, Cyclopentyl, Cyclobutyl, Cyclohexyl, Cycloheptyl oder Cyclooctyl. Bevorzugt seien genannt: Cyclopropyl, Cyclopentyl und Cyclohexyl.
Halogen steht für Fluor, Chlor, Brom und Jod. Bevorzugt sind Fluor, Chlor und Brom. Besonders bevorzugt sind Fluor und Chlor.
5- bis 6-gliedriges Heteroaryl steht für einen aromatischen Rest mit 5 bis 6 Ringatomen und bis zu 4 Heteroatomen aus der Reihe S, O und/oder N. Der Heteroarylrest kann über ein Kohlenstoff oder Heteroatom gebunden sein. Beispielsweise und vorzugsweise seien genannt: Thienyl, Furyl, Pyrrolyl, Thiazolyl, Oxazolyl, Imidazolyl, Tetrazolyl, Pyridyl, Pyrimidinyl, und Pyridazinyl.
4- bis 8-gliedriges Heterocyclyl steht für einen mono- oder polycyclischen, heterocyclischen Rest mit 4 bis 8 Ringatomen und bis zu 3, vorzugsweise 1 Heteroatomen bzw. Heterogruppen aus der Reihe N, O, S, SO, SO₂. Mono- oder bicyclisches Heterocyclyl ist bevorzugt. Besonders bevorzugt ist monocyclisches Carbocyclyl. Als Heteroatome sind N und O bevorzugt. Die Heterocyclyl-Reste können gesättigt oder teilweise ungesättigt sein. Gesättigte Heterocyclyl-Reste sind bevorzugt. Die Heterocyclyl-Reste können über ein Kohlenstoffatom oder ein Heteroatom gebunden sein. Besonders bevorzugt sind 5- bis 7-gliedrige, monocyclische gesättigte Heterocyclylreste mit bis zu zwei Heteroatomen aus der Reihe O, N und S. Beispielsweise und vorzugsweise seien genannt: Oxetan-3-yl, Pyrrolidin-2-yl, Pyrrolidin-3-yl, Pyrrolinyl, Tetrahydrofuranyl, Tetrahydrothienyl, Pyranyl, Piperidinyl, Thiopyranyl, Morpholinyl, Perhydroazepinyl.

Wenn Reste in den erfindungsgemäßen Verbindungen gegebenenfalls substituiert sind, können die Reste, soweit nicht anders spezifiziert, ein- oder mehrfach gleich oder verschieden substituiert sein. Eine Substitution mit bis zu drei gleichen oder verschiedenen Substituenten ist bevorzugt.

Bevorzugt sind Verbindungen der allgemeinen Formel (I),
in welcher
- R¹: für einen Aza-bicyclo[m.n.o]alkyl-Rest mit 7 bis 9 Ringatomen steht, worin m, n und o gleich oder verschieden sind und 0, 1, 2 oder 3 bedeuten, und wobei der Rest gegebenenfalls durch Methyl oder Ethyl substituiert ist,
und der Ring A, R² und R³ die oben angegebene Bedeutung haben.

Ebenfalls bevorzugt sind Verbindungen der allgemeinen Formel (I),
in welcher
der Ring A für Pyrido steht,
und R¹, R² und R³ die oben angegebene Bedeutung haben.

Besonders bevorzugt sind Verbindungen der allgemeinen Formel (I),
in welcher
der Ring A zusammen mit dem ankondensierten Benzolrest für Chinolin-6-yl steht, und R¹, R² und R³ die oben angegebene Bedeutung haben.

Ebenfalls bevorzugt sind Verbindungen der allgemeinen Formel (I),
in welcher
- R² und R³: gleich oder verschieden sind und für Reste ausgewählt aus der Gruppe Wasserstoff, Halogen, Cyano, Trifluormethyl, Methyl und Ethyl stehen,
und der Ring A und R¹ die oben angegebene Bedeutung haben.

Besonders bevorzugt sind Verbindungen der allgemeinen Formel (I),
in welcher
- R² und R³: gleich oder verschieden sind und für Reste ausgewählt aus der Gruppe Wasserstoff, Halogen, und Methyl stehen,
und der Ring A und R¹ die oben angegebene Bedeutung haben.

Ganz besonders bevorzugt sind Kombinationen von zwei oder mehreren der oben genannten Vorzugsbereiche.

Ebenfalls ganz besonders bevorzugt sind Verbindungen der allgemeinen Formel (I),
in welcher
- R¹: für 1-Aza-bicyclo[2.2.2]oct-3-yl steht,
der Ring A zusammen mit dem ankondensierten Benzolrest für Chinolin-5-yl oder Chinolin-6-yl steht, und
- R² und R³: gleich oder verschieden sind und für Reste ausgewählt aus der Gruppe Wasserstoff, Fluoro, Chloro, und Methyl stehen.

Die Erfindung betrifft weiterhin Verfahren zur Herstellung der Verbindungen der Formel (I), dadurch gekennzeichnet, dass man

Verbindungen der allgemeinen Formel (II),

R¹-NH₂ (II)

in welcher R¹ die oben genannte Bedeutung hat,
mit einer Verbindung der allgemeinen Formel (HI), 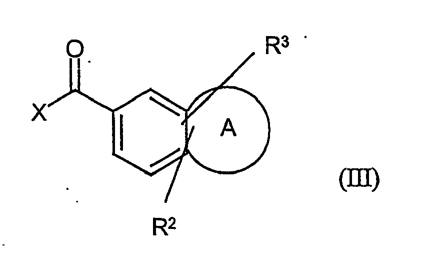 in welcher
der Ring A, R² und R³ die oben genannte Bedeutung haben, und
- X: für Hydroxy oder eine geeignete Abgangsgruppe steht,
in einem inerten Lösungsmittel gegebenenfalls in Gegenwart eines Kondensationsmittels und gegebenenfalls in Gegenwart einer Base umsetzt.

Wenn X eine Abgangsgruppe ist, sind Chloro, Mesyloxy und Isobutyloxycarbonyloxy, besonders Chloro bevorzugt.

Inerte Lösungsmittel sind beispielsweise Halogenkohlenwasserstoffe wie Methylenchlorid, Trichlormethan, Tetrachlormethan, Trichlorethan, Tetrachlorethan, 1,2-Dichlorethan oder Trichlorethylen, Ether wie Diethylether, Methyl-tert.-butylether, Dioxan, Tetrahydrofuran, Glykoldimethylether oder Diethylenglykoldimethylether, Kohlenwasserstoffe wie Benzol, Xylol, Toluol, Hexan, Cyclohexan oder Erdölfraktionen, oder andere Lösungsmittel wie Nitromethan, Ethylacetat, Aceton, Dimethylformamid, Dimethylacetamid, 1,2-Dimethoxyethan, Dimethylsulfoxid, Acetonitril oder Pyridin, bevorzugt sind Tetrahydrofuran, Dimethylformamid oder Chloroform.

Kondensationsmittel sind beispielsweise Carbodiimide wie z.B. N,N'-Diethyl-, N,N,'-Dipropyl-, N,N'-Diisopropyl-, N,N'-Dicyclohexylcarbodiimid, N-(3-Dimethylaminoisopropyl)-N'-ethylcarbodümid-Hydrochlorid (EDC), N-Cyclohexylcarbodiimid-N' - propyloxymethyl-Polystyrol (PS-Carbodiimid) oder Carbonylverbindungen wie Carbonyldümidazol, oder 1,2-Oxazoliumverbindungen wie 2-Ethyl-5-phenyl-1,2-oxazolium-3-sulfat oder 2-tert.-Butyl-5-methyl-isoxazolium-perchlorat, oder Acylaminoverbindungen wie 2-Ethoxy-1-ethoxycarbonyl-1,2-dihydrochinolin, oder Propanphosphonsäureanhydrid, oder Isobutylchloroformat, oder Bis-(2-oxo-3-oxazolidinyl)-phosphorylchlorid oder Benzotriazolyloxy-tri(dimethylamino)phosphoniumhexafluorophosphat, oder O-(Benzotriazol-1-yl)-N,N,N',N'-tetra-methyluronium-hexafluorophosphat (HBTU), 2-(2-Oxo-1-(2H)-pyridyl)-1,1,3,3-tetramethyluroniumtetrafluoro-borat (TPTU) oder O-(7-Azabenzotriazol-1-yl)-N,N,N',N'-tetramethyl-uroniumhexafluorophosphat (HATU) oder Benzotriazol-1-yloxy-tris(dimethylamino)-phosphoniumhexafluoro-phosphat (BOP), oder Mischungen aus diesen.

Gegebenenfalls kann es vorteilhaft sein, diese Kondensationsmittel in Gegenwart eines Hilfsnucleophils wie z.B. 1-Hydroxybenztriazol (HOBt) zu verwenden.

Basen sind beispielsweise Alkalicarbonate, wie z.B. Natrium- oder Kaliumcarbonat, oder -hydrogencarbonat, oder organische Basen wie Trialkylamine z.B. Triethylamin, N-Methylmorpholin, N-Methylpiperidin, 4- Dimethylaminopyridin oder Düsopropylethylamin.

Besonders bevorzugt ist die Kombination von N-(3-Dimethylaminoisopropyl)-N'-ethylcarbodiimid-Hydrochlorid (EDC) und 1-Hydroxybenztriazol (HOBt) in Dimethylformamid.

Vorzugsweise wird das erfindungsgemäße Verfahren in einem Temperaturbereich von Raumtemperatur bis 50°C bei Normaldruck durchgeführt.

Die Verbindungen der allgemeinen Formeln (II) und (III) sind bekannt oder lassen sich nach bekannten Verfahren aus den entsprechenden Edukten synthetisieren (vgl. z.B., Comprehensive Heterocyclic Chemistry', Katritzki et al., Hrsg.; Elsevier, 1996).

So können beispielsweise Chinolincarbonsäuren [X¹ steht für Hydroxy und der Ring A für [b]Pyrido in Verbindungen der allgemeinen Formel (III)], durch Oxidation der entsprechenden Methylchinoline (Miller et al. Chem.Ber. 1890, 23, 2263 ff.) oder der entsprechenden Aldehyde (Howitz et al. Justus Liebigs Ann. Chem. 1913, 396, 37) mit geeigneten Oxidationsmitteln wie z.B. Cr₂O₃ erhalten werden.

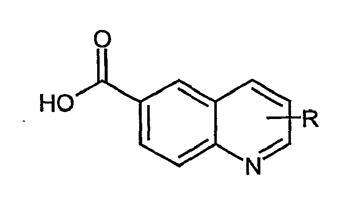

Weiterhin kann man beispielsweise 6-Chinolincarbonsäuren durch Umsetzung von 4-Aminobenzoesäure mit geeigneten Enonen erhalten (John et al J.Prakt.Chem. 1925, 111, 95), wie durch das folgende Syntheseschema beispielhaft verdeutlicht wird.

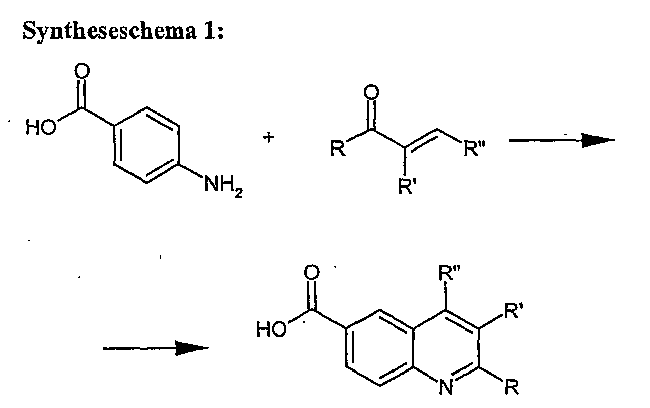

Alternativ können erfindungsgemäße Chinolincarbonsäureamide hergestellt werden, indem man die entsprechenden Iod- und Aminosubstituierten Benzoesäureamide in der Gegenwart von geeigneten Katalysatoren mit Allyl- und Propargylalkoholen umsetzt (vgl. Kuo et al. Tetrahedron Lett. 1991, 32, 569), wie durch das folgende Syntheseschema beispielhaft verdeutlicht wird.

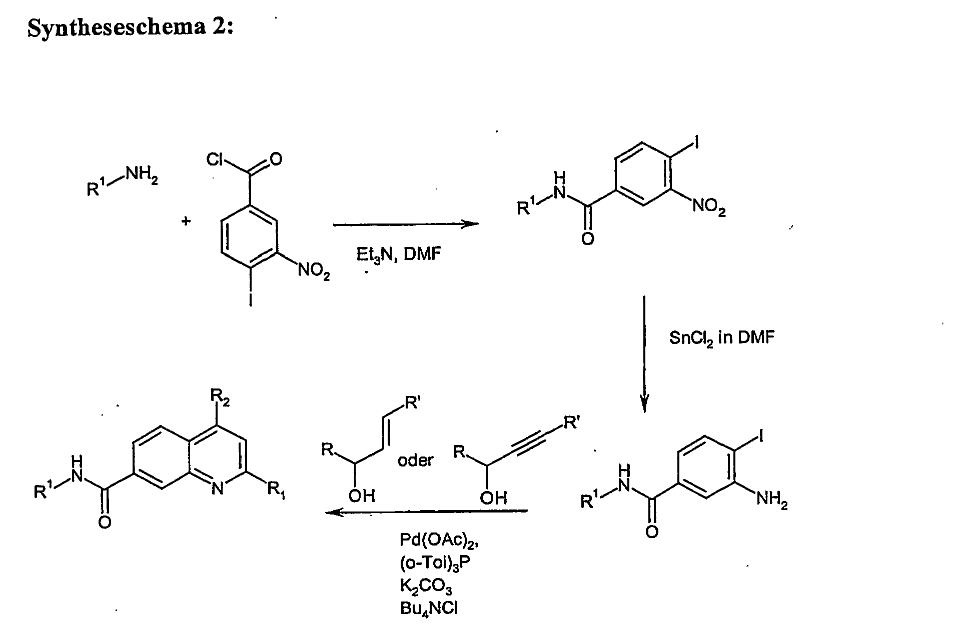

Die erfindungsgemäßen Verbindungen der allgemeinen Formel (I) eignen sich zur Verwendung als Arzneimittel zur Behandlung und/oder Prophylaxe von Krankheiten bei Menschen und/oder Tieren.

Die erfindungsgemäßen Verbindungen zeigen ein nicht vorhersehbares, wertvolles pharmakologisches Wirkspektrum.

Sie zeichnen sich als Liganden, insbesondere Agonisten am α7-nAChR aus.

Die erfindungsgemäßen Verbindungen können aufgrund ihrer pharmakologischen Eigenschaften allein oder in Kombination mit anderen Arzneimitteln zur Behandlung und/oder Prävention von kognitiven Störungen , insbesondere der Alzheimerschen Krankheit eingesetzt werden. Wegen ihrer selektiven Wirkung als α7-nAChR Agonisten eignen sich die erfindungsgemäßen Verbindungen besonders zur Verbesserung der Wahrnehmung, Konzentrationsleistung, Lernleistung, oder Gedächtnisleistung insbesondere nach kognitiven Störungen, wie sie beispielsweise bei Situationen/Krankheiten/Syndromen auftreten wie "Mild cognitive impairment", Altersassoziierte Lern- und Gedächtnisstörungen, Altersassoziierte Gedächtnisverluste, Vaskuläre Demenz, Schädel-Hirn-Trauma, Schlaganfall, Demenz, die nach Schlaganfällen auftritt ("post stroke dementia"), post-traumatisches Schädel Hirn Trauma, allgemeine Konzentrationsstörungen, Konzentrationsstörungen in Kindern mit Lern- und Gedächtnisproblemen, Attention Deficit Hyperactivity Disorder, Alzheimersche Krankheit, Vaskuläre Demenz, Demenz mit Lewy-Körperchen, Demenz mit Degeneration der Frontallappen einschließlich des Pick's Syndroms, Parkinsonsche Krankheit, Progressive nuclear palsy, Demenz mit corticobasaler Degeneration, Amyotrophe Lateralsklerose (ALS), Huntingtonsche Krankheit, Multiple Sklerose, Thalamische Degeneration, Creutzfeld-Jacob-Demenz, HIV-Demenz, Schizophrenie, Schizophrenie mit Demenz oder Korsakoff-Psychose.

Die erfindungsgemäßen Verbindungen können allein oder in Kombination mit anderen Arzneimitteln eingesetzt werden zur Prophylaxe und Behandlung von akuten und/oder chronischen Schmerzen (für eine Klassifizierung siehe "Classification of Chronic Pain, Descriptions of Chronic Pain Syndromes and Definitions of Pain Terms", 2. Aufl., Meskey und Begduk, Hrsg.; LASP-Press, Seattle, 1994), insbesondere zur Behandlung von Krebs-induzierten Schmerzen und chronischen neuropathischen Schmerzen, wie zum Beispiel, bei diabetischer Neuropathie, postherpetischer Neuralgie, peripheren Nervenbeschädigungen, zentralem Schmerz (beispielsweise als Folge von cerebraler Ischämie) und trigeminaler Neuralgie, und anderen chronischen Schmerzen, wie zum Beispiel Lumbago, Rückenschmerz (low back pain) oder rheumatischen Schmerzen. Daneben eignen sich diese Substanzen auch zur Therapie von primär akuten Schmerzen jeglicher Genese und von daraus resultierenden sekundären Schmerzzuständen, sowie zur Therapie chronifizierter, ehemals akuter Schmerzzustände.

Die *in vitro-*Wirkung der erfindungsgemäßen Verbindungen kann in folgenden Assays gezeigt werden:

### 1. Bestimmung der Affinität von Testsubstanzen für α7-nAChR durch Inhibition von [³H]Methyllycaconitine-Bindung an Rattenhirnmembranen

Der [³H]-Methyllycaconitine Bindungstest ist eine Modifikation der von Davies et al. (Neuropharmacol. 1999, 38, 679-690) beschriebenen Methode.

Rattenhirngewebe (Hippocampus oder Gesamthirn) wird in Homogenisierungspuffer (10 % w/v) (0.32 M Sucrose, 1 mM EDTA, 0.1 mM Phenylmethylsulfonyl fluorid (PMSF), 0.01 % (w/v) NaN₃, pH 7.4, 4 °C) bei 600 rpm in einem Glashomogenisator homogenisiert. Das Homogenat wird zentrifugiert (1000 x g, 4°C, 10 min) und der Überstand wird abgenommen. Das Pellet wird erneut suspendiert (20 % w/v) und zentrifugiert (1000 x g, 4°C, 10 min). Die beiden Überstände werden vereinigt und zentrifugiert (15.000 x g, 4°C, 30 min). Dieses Pellet wird als P2 Fraktion bezeichnet.

Das P2-Pellet wird zweimal mit Bindungspuffer gewaschen (50 mM Tris-HCI, 1 mM MgCl₂, 120 mM NaCl, 5 mM KCl, 2 mM CaCl₂, pH 7.4) und zentrifugiert (15.000 x g, 4°C, 30 min).

Die P2 Membranen werden in Bindungspuffer resuspendiert und in einem Volumen von 250µl (Membranproteinmenge 0.1 - 0.5 mg) für 2.5 h bei 21°C inkubiert in der Gegenwart von 1-5 nM [³H]-Methyllycaconitine, 0.1 % (w/v) BSA (bovines Serumalbumin) und verschiedenen Konzentrationen der Testsubstanz. Die unspezifische Bindung wird bestimmt durch Inkubation in der Gegenwart von 1 µM α-Bungarotoxin oder 100 µM nicotine oder 10 µM MLA (Methyllycaconitine).

Die Inkubation wird beendet durch Zugabe von 4 ml PBS (20 mM Na₂HPO₄, 5 mM KH₂PO₄, 150 mM NaCl, pH 7.4, 4°C) und Filtration durch Typ A/E glass fibre filters (Gelman Sciences), die vorher 3 h in 0.3 % (v/v) Polyethyleneimine (PEI) eingelegt waren. Die Filter werden zweimal mit 4 ml PBS (4°C) gewaschen und die gebundene Radioaktivität durch Szintillationsmessung bestimmt. Alle Tests werden in Dreifachbestimmungen durchgeführt. Aus dem IC₅₀-Wert der Verbindungen (Konzentration der Testsubstanz, bei der 50% des am Rezeptor gebundenen Liganden verdrängt werden), der Dissoziationskonstante K_{D} und der Konzentration L von [³H]Methyllycaconitine wurde die Dissoziationskonstante der Testsubstanz Kᵢ bestimmt (Kᵢ = IC₅₀ / (1+L/K_{D})).

Anstelle von [³H]-Methyllycaconitine können auch andere α7-nAChR-selektive Radioliganden wie z.B. [¹²⁵I]-α-Bungarotoxin oder unselektive nAChR-Radioliganden gemeinsam mit Inhibitoren anderer nAChR eingesetzt werden.

Die Eignung der erfindungsgemäßen Verbindungen zur Behandlung von kognitiven Störungen kann in folgenden Tiermodellen gezeigt werden:

### 2. Objekt-Wiedererkennungstest

Der Objekt-Wiedererkennungstest ist ein Gedächtnistest. Er misst die Fähigkeit von Ratten (und Mäusen), zwischen bekannten und unbekannten Objekten zu unterscheiden.

Der Test wird wie beschrieben durchgeführt (Blokland et al. NeuroReport 1998, 9, 4205-4208; Ennaceur, A., Delacour, J.,. Behav. Brain Res. 1988, 31, 47-59; Ennaceur, A., Meliani, K.,. Psychopharmacology 1992, 109, 321-330; Prickaerts, et al. Eur. J. Pharmacol. 1997, 337, 125-136).

In einem ersten Durchgang wird eine Ratte in einer ansonsten leeren größeren Beobachtungsarena mit zwei identischen Objekten konfrontiert. Die Ratte wird beide Objekte ausgiebig untersuchen, d.h. beschnüffeln und berühren. In einem zweiten Durchgang, nach einer Wartezeit von 24 Stunden, wird die Ratte erneut in die Beobachtrungsarena gesetzt. Nun ist eines der bekannten Objekte durch ein neues, unbekanntes Objekt ersetzt. Wenn eine Ratte das bekannte Objekt wiedererkennt, wird sie vor allem das unbekannte Objekt untersuchen. Nach 24 Stunden hat eine Ratte jedoch normalerweise vergessen, welches Objekt sie bereits im ersten Durchgang untersucht hat, und wird daher beide Objekte gleichstark inspektieren. Die Gabe einer Substanz mit lern-und gedächtnisverbessernder Wirkung wird dazu führen, dass eine Ratte das bereits 24 Stunden vorher, im ersten Durchgang, gesehene Objekt als bekannt wiedererkennt. Sie wird das neue, unbekannte Objekt ausführlicher untersuchen als das bereits bekannte. Diese Gedächtnisleistung wird in einem Diskriminationsindex ausgedrückt. Ein Diskiminationsindex von Null bedeutet, dass die Ratte beide Objekte, das alte und das neue, gleichlang untersucht; d.h. sie hat das alte Objekt nicht wiedererkannt und reagiert auf beide Objekte als wären sie unbekannt und neu. Ein Diskriminationsindex größer Null bedeutet, dass die Ratte das neue Objekt länger inspektiert als das alte; d.h. die Ratte hat das alte Objekt wiedererkannt.

### 3. Sozialer Wiedererkennungstest:

Der Soziale Wiedererkennungstest ist ein Test zur Prüfung der lern- oder gedächtnisverbessernden Wirkung von Testsubstanzen.

Erwachsene Ratten, die in Gruppen gehalten werden, werden 30 Minuten vor Testbeginn einzeln in Testkäfige gesetzt. Vier Minuten vor Testbeginn wird das Testtier in eine Beobachtungsbox gebracht. Nach dieser Adaptationszeit wird ein juveniles Tier zu dem Testtier gesetzt und 2 Minuten lang die totale Zeit gemessen, die das adulte Tier das Junge investigiert (Trial 1). Gemessen werden alle deutlich auf das Jungtier gerichteten Verhaltensweisen, d.h. ano-genitale Inspektion, Verfolgen sowie Fellpflege, bei denen das Alttier einen Abstand von höchstens 1 cm zu dem Jungtier hat. Danach wird das Juvenile herausgenommen und das Adulte in seinem Testkäfig belassen (bei 24 Stunden Retention wird das Tier in seinen Heimkäfig zurückgesetzt). Vor oder nach dem ersten Test wird das Versuchstier mit Substanz behandelt. Je nach Zeitpunkt der Substanzgabe wird das Erlernen oder das Speichern der Information über das Jungtier durch die Substanz beeinflusst. Nach einem festgelegten Zeitraum (Retention) wird der Test wiederholt (Trial 2). Je größer die Differenz zwischen den in Trial 1 und 2 ermittelten Investigationszeiten, desto besser hat sich das adulte Tier an das Jungtier erinnert

Die erfindungsgemäßen Verbindungen der allgemeinen Formel (I) eignen sich zur Verwendung als Arzneimittel für Menschen und Tiere.

Zur vorliegenden Erfindung gehören auch pharmazeutische Zubereitungen, die neben inerten, nicht-toxischen, pharmazeutisch geeigneten Hilfs- und Trägerstoffen eine oder mehrere Verbindungen der allgemeinen Formel (I) enthalten, oder die aus einem oder mehreren Verbindungen der Formel (I) bestehen, sowie Verfahren zur Herstellung dieser Zubereitungen.

Die Verbindungen der Formel (I) sollen in diesen Zubereitungen in einer Konzentration von 0,1 bis 99,5 Gew.-%, bevorzugt von 0,5 bis 95 Gew.-% der Gesamtmischung vorhanden sein.

Neben den Verbindungen der Formel (I) können die pharmazeutischen Zubereitungen auch andere pharmazeutische Wirkstoffe enthalten.

Die oben aufgeführten pharmazeutischen Zubereitungen können in üblicher Weise nach bekannten Methoden hergestellt werden, beispielsweise mit dem oder den Hilfs-oder Trägerstoffen.

Die neuen Wirkstoffe können in bekannter Weise in die üblichen Formulierungen überführt werden, wie Tabletten, Dragees, Pillen, Granulate, Aerosole, Sirupe, Emulsionen, Suspensionen und Lösungen, unter Verwendung inerter, nicht toxischer, pharmazeutisch geeigneter Trägerstoffe oder Lösungsmittel. Hierbei soll die therapeutisch wirksame Verbindung jeweils in einer Konzentration von etwa 0,5 bis 90 Gew.-% der Gesamtmischung vorhanden sein, d.h. in Mengen, die ausreichend sind, um den angegebenen Dosierungsspielraum zu erreichen.

Die Formulierungen werden beispielsweise hergestellt durch Verstrecken der Wirkstoffe mit Lösungsmitteln und/oder Trägerstoffen, gegebenenfalls unter Verwendung von Emulgiermitteln und/oder Dispergiermitteln, wobei z.B. im Fall der Benutzung von Wasser als Verdünnungsmittel gegebenenfalls organische Lösungsmittel als Hilfslösungsmittel verwendet werden können.

Die Applikation erfolgt in üblicher Weise, vorzugsweise oral, transdermal oder parenteral, insbesondere perlingual oder intravenös. Sie kann aber auch durch Inhalation über Mund oder Nase, beispielsweise mit Hilfe eines Sprays erfolgen, oder topisch über die Haut.

Im Allgemeinen hat es sich als vorteilhaft erwiesen, Mengen von etwa 0,001 bis 10 mg/kg, bei oraler Anwendung vorzugsweise etwa 0,005 bis 3 mg/kg Körpergewicht zur Erzielung wirksamer Ergebnisse zu verabreichen.

Trotzdem kann es gegebenenfalls erforderlich sein, von den genannten Mengen abzuweichen, und zwar in Abhängigkeit vom Körpergewicht bzw. der Art des Applikationsweges, vom individuellen Verhalten gegenüber dem Medikament, der Art von dessen Formulierung und dem Zeitpunkt bzw. Intervall, zu welchen die Verabreichung erfolgt. So kann es in einigen Fällen ausreichend sein, mit weniger als der vorgenannten Mindestmenge auszukommen, während in anderen Fällen die genannte obere Grenze überschritten werden muss. Im Falle der Applikation größerer Mengen kann es empfehlenswert sein, diese in mehreren Einzelgaben über den Tag zu verteilen.

### Abkürzungen:

- DMF: *N,N*-Dimethylformamid
- DMSO: Dimethylsulfoxid
- HOBt: 1-Hydroxy-1H-benzotriazol x H₂O
- NMR: Kernresonanzspektroskopie
- RT: Raumtemperatur
- TBTU: *O*-(Benzotriazol-1-yl)-*N,N,N',N'*-tetramethyluronium-Tetrafluoroborat
- THF: Tetrahydrofuran

### Ausgangsverbindungen

### Beispiel 1A

### 4-Amino-3-Iodbenzoesäure

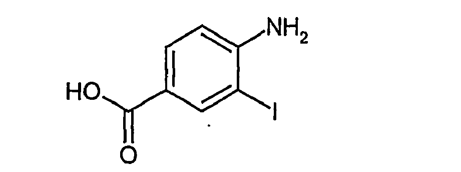

Eine Lösung von 3,00 g (10,8 mmol) 4-Amino-3-Iodbenzoesäuremethylester und 1,30 g (54,1 mmol) Lithiumhydroxid in 150 ml eines Gemisches von Dioxan/Wasser (1:1) wurde 6 h bei Raumtemperatur gerührt. Das Dioxan wurde im Vakuum abdestilliert und die verbleibende wässrige Phase wurde mit 1 M Salzsäure auf pH 5 gestellt. Der Niederschlag wurde abgesaugt und mit Wasser gewaschen. Man erhielt 2,80 g (98 %) der Titelverbindung als Feststoff.

HPLC (Kromasil RP-18, 60x2,1 mm, Eluent = A: H₂O + 5 mL HClO₄/L; B: Acetonitril; Gradient = 0 - 4.5 min 98 % A - 90 % B; 4.5 - 6.5 min 90 % B; 0.75 mL/min; Temp.: 30°C, UV-Detektion bei 210 nm): Rt = 3.51 min
¹H-NMR (300 MHz in D₆-DMSO) δ = 5,98 (s, 2H), 6,74 (d, 1H), 7,63 (m, 1H), 8,10 (s, 1H), 12,33 (s, breit, 1H)
MS (ESI+): m/z = 281 [M+NH₄]⁺

### Beispiel 2A

### 4-Amino-N-(1-azabicyclo[2.2.2]oct-3-yl)-3-iodbenzamid

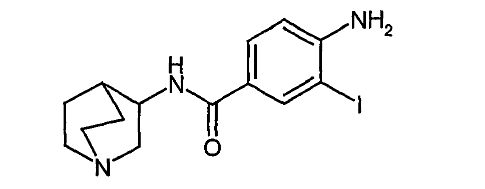

Eine Lösung von 2,04 g (10,3 mmol) 3-Aminoquinuclidindihydrochlorid, 2,70 g (10,3 mmol) 4-Amino-3-Iodbenzoesäure, 1,39 g ( 10,3 mmol) HOBt, 2,16 g (11,3 mmol) EDC und 6,63 g (51,3 mmol) N,N-Diisopropylethylamin in 150 ml DMF wurde für 16 h bei Raumtemperatur gerührt. Es wurde mit 300 ml Wasser versetzt und die wässrige Pahse wurde dreimal mit je 300 ml Essigsäureethylester extrahiert. Die vereinigten organischen Phasen wurden über Natriumsulfat getrocknet und das Lösungsmittel wurde im Vakuum abdestilliert. Der Rückstand wurde über eine Flash-Säule gereinigt (Laufmittel: Dichlormethan / Methanol / Triethylamin 90:10:0.2). Man erhielt 3,45 g (87 %) der gewünschten Titelverbindung.

HPLC (Bedingungen wie bei Beispiel 1A): Rt = 3,29 min
¹H-NMR (200 MHz in D₆-DMSO) δ = 1,18-1,87 (m, 5H), 2,58-3,13 (m, 6H), 3,49 (m, breit, 1H), 3,89 (m, 1H), 5,75 (m, 3H), 6,72 (d, 1H), 7,63 (dd, 11-1), 7,92 (d, 1H), 8,12 (d, 1H)
MS (ESI+): m/z = 372 [M+H]⁺

### Beispiel 3A

### N-(1-Azabicyclo[2.2.2] oct-3-yl)-4-iod-3-nitrobenzamid

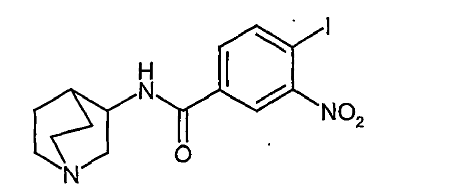

Eine Lösung von 3,08 g ( 24,4 mmol) 3-Aminoquinuclidindihydrochlorid, 7,60 g (24,4 mmol) 4-Iod-3-nitrobenzoylchlorid, 9.88 g (97.6 mmol) Triethylamin in 150 ml DMF wurde über Nacht bei Raumtemperatur gerührt. Das Lösungsmittel wurde am Rotationsverdampfer abdestilliert, es wurde in 200 ml Dichlormethan auf genommen und mit 200 ml einer ges. Natriumhydrogencarbonat-Lsg. versetzt. Die organische Phase wurde über Natriumsulfat getrocknet und das Lösungsmittel wurde: im Vakuum abdestilliert. Man erhielt 2,20 g (22,5 %) der gewünschten Titelverbindung.
HPLC (Bedingungen wie bei Beispiel 1A): Rt = 3.72 min.
¹H-NMR (300 MHz in CDCl₃) δ = 1,24-1,78 (m, 5H), 2,05 (m, 1H), 2,61 (m, 1H), 2,89 (m, 3H), 3,46 (m, 1H), 4,15 (m, 1H), 6,24 (m, 1H), 7,67 (dd, 1H), 8,14 (d, 1H), 8,20 (d, 1H).
MS (ESI+): m/z = 402 [M+H]⁺

### Beispiel 4A

### 3-Amino-N-(1-azabicyclo[2.2.2]oct-3-yl)-4-iodbenzamid

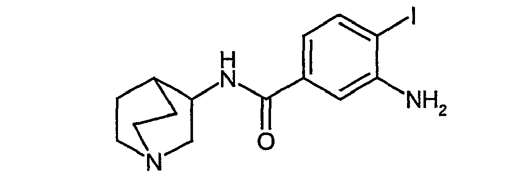

Eine Lösung von 100 mg (0,25 mmol) N-(1-Azabicyclo[2.2.2]oct-3-yl)-4-iod-3-nitrobenzamid und 281mg (1,25 mmol) Zinn(II)chloriddihydrat in 5 ml DMF wurde 6 h bei Raumtemperatur gerührt. Das Lösungsmittel wurde am Rotationsverdampfer abdestilliert, der Rückstand wurde in 10 ml Dichlormethan aufgenommen und mit 10 ml einer wässrigen 1 M Natronlauge versetzt. Die organische Phase wurde über Natriumsulfat getrocknet und das Lösungsmittel wurde am Rotationsverdampfer abdestilliert. Man erhielt 91 mg (98 %) der gewünschten Titelverbindung.

HPLC (Bedingungen wie bei Beispiel 1A): Rt = 3,28 min.
¹H-NMR (300 MHz in D₆-DMSO) δ = 1,21-1,85 (m, 5H), 2,64 (m, 4H), 2,85 (m, 1H), 3,05 (m, 1H), 3,89 (m, 1H), 5,34 (s, breit, 2H), 6,78 (dd, 1H), 7,15 (m, 1H), 7,60 (d, 1H), 8,13 (d, 1H).
MS (ESI+): m/z = 372 [M+H]⁺

### Ausführungsbeispiele:

### Beispiel 1

### N-(1-Azabicyclo[2.2.2]oct-3-yl)-6-chinolincarboxamid Hydrochlorid

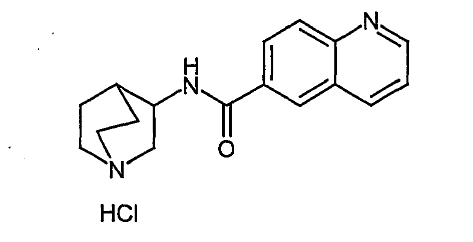

Zu einer Lösung von 182 mg (1,05 mmol) Chinolin-6-carbonsäure und Diisopropylethylamin (620 mg, 4,8 mmol) in 4mL DMF wird bei RT zunächst 319 mg (0,99 mmol) TBTU und 137 mg (1,01 mmol) HOBt gegeben, anschließend wird 200 mg (1,0 mmol) 3-Aminochinuclidindihydrochlorid zugegeben. Das Gemisch wird 4h bei RT gerührt. Zur Aufarbeitung wird eingeengt und in einer Mischung aus Chloroform und überschüssiger wässriger NaOH aufgenommen. Die Phasen werden getrennt und die wässrige Phase mehrmals mit Chloroform nachextrahiert. Die vereinigten organischen Phasen werden über Natriumsulfat getrocknet, eingeengt und das Rohprodukt an Kieselgel gesäult (Laufmittel, Chloroform:Methanol: konz. NH₃ = 100:20:4). Das erhaltene Produkt wird in THF aufgenommen, mit überschüssigem HCI in Diethylether versetzt, eingeengt und am Hochvakuum getrocknet. Man erhält 136 mg (47% Ausbeute) des Hydrochlorids.
¹H-NMR (300 MHz, CD₃OD) δ = 9,30 (m, 2H); 8,95 (s, 1H); 8,60 (d, 1H), 8,32 (d, 1H); 8,15 (m, 1H); 4,55 (m, 1H), 3,85 (m, 1H); 3,60-3,30 (m, 5H); 2,50-1,90 (m, 5H).
MS (ESI+): m/z = 282 ([M+H]⁺ der freien Base)

### Beispiel 2

### N-(1-Azabicyclo[2.2.2]oct-3-yl)-2-phenazincarboxamid

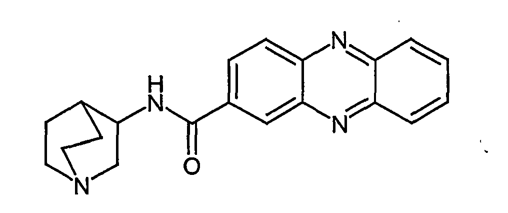

Hergestellt analog der Arbeitsvorschrift für Beispiel 1 ausgehend von 236 mg Phenazin-2-carbonsäure und 200 mg 3-Aminochinuclidindihydrochlorid. Nach chromatographischer Trennung wurden 84 mg (25 % Ausbeute) der freien Base erhalten.
¹H-NMR (in DMSO-d₆) δ = 8,85 (s, 1H); 8,75 (d, J = 7Hz, 1H); 8,30 (m, 4H); 8,00 (m, 2H); 4,05 (m, 1H); 3,2-2,7 (m, 6H); 2,0-1,3 (m, 5H).
MS (ESI+): m/z = 333 [M+H]⁺

### Beispiel 3

### N-(1-Azabicyclo[2.2.2]oct-3-yl)-7-chinolincarboxamid Hydrochlorid

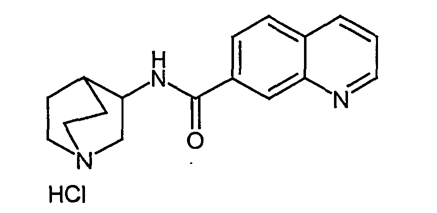

Hergestellt analog der Arbeitsvorschrift für Beispiel 1 ausgehend von 183 mg Chinolin-7-carbonsäure (Seibert et al. J. Anz. Chem. Soc. 1946, 68, 2721) und 200 mg 3-Aminochinuclidindihydrochlorid. Es wurden 227 mg (71 % Ausbeute) des Hydrochlorids erhalten.
¹H-NMR (300 MHz in DMSO-d₆) δ = 10,5 (s, 1H); 9,15 (m, 2H); 8,70 (m, 2H), 8,20 (m, 2H); 7,80 (m, 1H); 4,40 (m, 1H); 3,65 (m, 1H), 3,45-3,10 (m, 5H); 2,30-1,65 (m, 5H).
MS (ESI+): m/z = 282 ([M+H]⁺ der freien Base)

### Beispiel 4

### N-[(3R)-1-Azabicyclo[2.2.2]oct-3-yl]-6-chinolincarboxamid Hydrochlorid

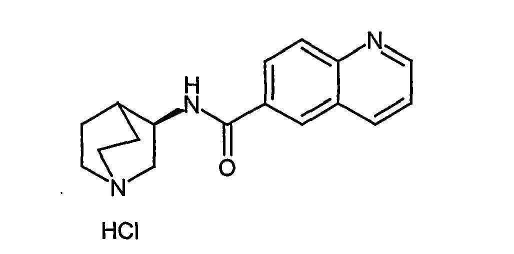

Hergestellt analog Beispiel 1 ausgehend von (3R)-1-Azabicyclo[2.2.2]oct-3-ylamin. Die ¹H-NMR und MS-Daten waren identisch zu Beispiel 1.

### Beispiel 5

### N-(1-Azabicyclo[2.2.2]oct-3-yl)-2-ethyl-7-chinolincarboxamid

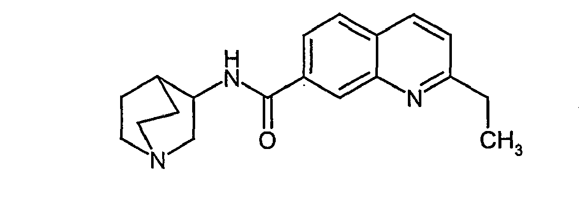

Eine Lösung von 30 mg (0,08 mmol) des Beispiels 4A, 0,32 mmol 1-Penten-3-ol, 0,5 mg (10 mol%) Palladium(II)acetat, 22,4 mg (0,08 mmol) Tetrabutylammoniumchlorid, 0,6 mg (10 mol%) Tri-tertbutylphosphin und 28 mg (0,20 mmol) Kaliumcarbonat in 2 ml DMF wurde in einer Argonatmosphäre 72 h bei 100°C gerührt. Das Lösungsmittel wurde abdestilliert und der Rückstand wurde in Methanol aufgenommen. Es wurde über Dickschicht-Chromatographie gereinigt (Laufmittel: Dichlormethan / Methanol / Triethylamin 80:20:2).
Man erhielt die Titelverbindung in 12 % Ausbeute.

HPLC (Bedingungen wie bei Beispiel 1A): Rt = 3,00 min.
¹H-NMR (400 MHz in D₆-DMSO) δ = 1,35 (t, 3H), 1,21-2,01 (m, 5H), 2,96 (q, 2H), 2,82-3,12 (m, 5H), 4,11 (m, 1H), 7,52 (d, 1H), 7,95 (m, 2H), 8,31 (d, 1H), 8,54 (s, 1H), 8,64 (d, 1H).
MS (ESI+): m/z = 310 [M+H]⁺

### Beispiel 6

### N-(1-Azabicyclo[2.2.2]oct-3-yl)-2-ethyl-6-chinolincarboxamid

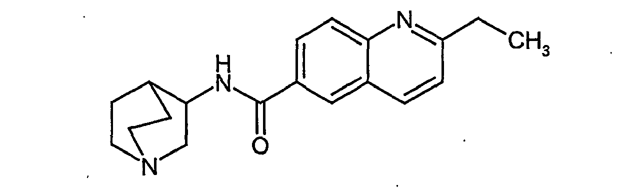

In Analogie zur Vorschrift für Beispiel 5 wurde Beispiel 2A mit 1-Penten-3-ol umgesetzt. Man erhielt die Titelverbindung in 36 % Ausbeute.
LC/MS (Kromasil RP-18, 5 µm, 2,1x150 mm, Eluent = A: Acetonitril B: H₂O + 0,23 g 30%ige HCl/l Wasser; Gradient =0-2,5 min 2 % A - 95 % A; 2,5 - 5 min 95 % A; Fluß = 0,9 mL/min; Temp.: 70°C, UV Detektion bei 210 nm): Rt = 1,71 min; MS (ESI+): m/z=310 [M+H]⁺.
¹H-NMR (400 MHz in D₆-DMSO) δ = 1.32 (t, 3H), 1.25-2.22 (m, 5H), 2.76 (m,4H), 2.97 (m, 5H), 4.06 (m, 1H), 7.52 (d, 1H), 7.98 (d, 1), 8.12 (dd, 1H), 8.36 (d, 1H), 8.43 (s, 1H), 8.52 (d, 1H).

### Beispiel 7

### N-(1-Azabicyclo[2.2.2]oct-3-yl)-2-methyl-7-chinolincarboxamid

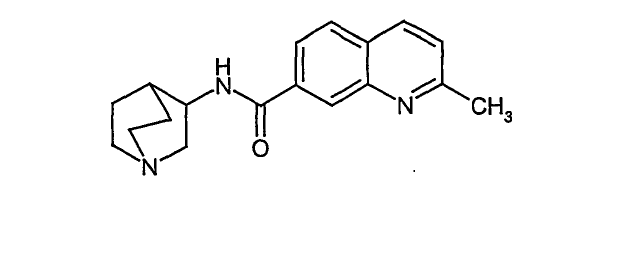

In Analogie zur Vorschrift für Beispiel 5 wurde Beispiel 4A mit 1-Buten-3-ol umgesetzt. Man erhielt die Titelverbindung in 21 % Ausbeute.

LC/MS (Bedingungen wie bei Beispiel 6): Rt = 1,70 min; MS (ESI+): m/z = 296 [M+H]⁺.
¹H-NMR (400 MHz in D₆-DMSO) δ = 1,38-1,99 (m, 5R), 2,68 (s, 3H), 2,82 (m, 4H), 3,01 (m, 1H), 4,10 (m, 1H), 7,50 (d, 1H), 7,96 (m, 2H), 8,30 (d, 1H), 8,51 (s, 1H), 8,60 (s, 1H).

### Beispiel 8

### N-(1-Azabicyclo[2.2.2]oct-3-yl)-2-methyl-6-chinolincarboxamid

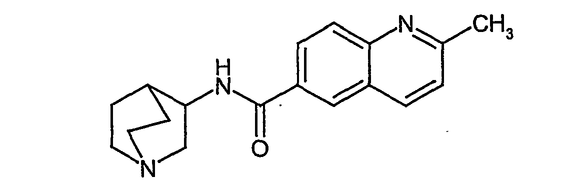

In Analogie zur Vorschrift für Beispiel 5 wurde Beispiel 2A mit 1-Buten-3-ol umgesetzt. Man erhielt die Titelverbindung in 29 % Ausbeute.

LC/MS (Bedingungen wie bei Beispiel 6): Rt = 0,57 min; MS (ESI+): m/z = 296 [M+H]⁺.

### Beispiel 9

### N-(1-Azabicyclo[2.2.2]oct-3-yl)-4-methyl-6-chinolincarboxamid

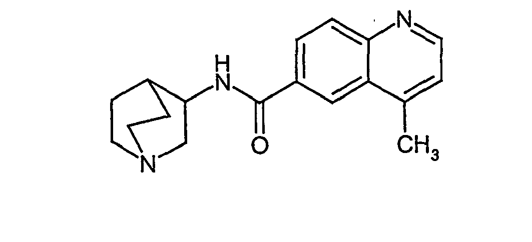

In Analogie zur Vorschrift für Beispiel 5 wurde Beispiel 2A mit 2-Butin-1-ol umgesetzt. Man erhielt die Titelverbindung in 8 % Ausbeute.

LC/MS (Bedingungen wie bei Beispiel 6): Rt = 0,56 min; MS (ESI+): m/z = 296 [M+H]⁺.

### Beispiel 10

### N-(1-Azabicyclo[2.2.2]oct-3-yl)-2-propyl-6-chinolincarboxamid

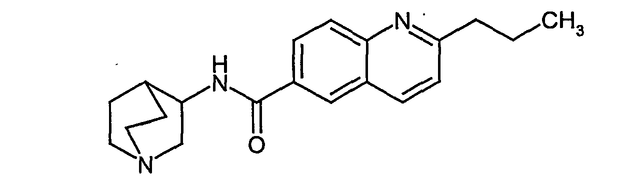

In Analogie zur Vorschrift für Beispiel 5 wurde Beispiel 2A mit 1-Hexen-3-ol umgesetzt. Man erhielt die Titelverbindung in 23 % Ausbeute.

LC/MS (Bedingungen wie bei Beispiel 6): Rt = 1,77 min; MS (ESI+): m/z = 324 [M+H]⁺.
¹H-NMR (400 MHz in D₆-DMSO) δ = 0,95 (t, 3H), 1,24-1,65 (m, 4H), 1,85 (m, 4H), 2,70 (m, 3H), 2,91 (m, 3H), 3,15 (m, 1H), 4,01 (m, 1H), 5,76 (m, 1H), 7,50 (d, 1H), 7,96 (m, 1H), 8,12 (d, 1H), 8,38 (d, 1H), 8,47 (m, 2H).

### Beispiel 11

### N-(1-Azabicyclo[2.2.2]oct-3-yl)-2-ethyl-4-methyl-6-chinolincarboxamid

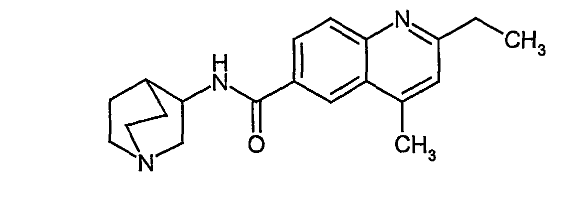

In Analogie zur Vorschrift für Beispiel 5 wurde Beispiel 2A mit 2-Hexin-4-ol umgesetzt. Man erhielt die Titelverbindung in 16 % Ausbeute.

LC/MS (Bedingungen wie bei Beispiel 6): Rt = 2,06 min; MS (ESI+): m/z = 324 [M+H]⁺.
¹H-NMR (400 MHz in D₆-DMSO) δ = 1,31 (t, 3H), 1,61 (m, 2H), 1,90 (m, 2H), 2,71 (m, 8H), 2,91 (m, 4H), 3,17 (m, 1H), 4,01 (m, 1H), 7,39 (s, 1H), 7,95 (d, 1H), 8,13 (m, 1H), 8,49 (m, 2H).

### Beispiel 12

### N-(1-Azabicyclo[2.2.2]oct-3-yl)-2-propyl-7-chinolincarboxamid

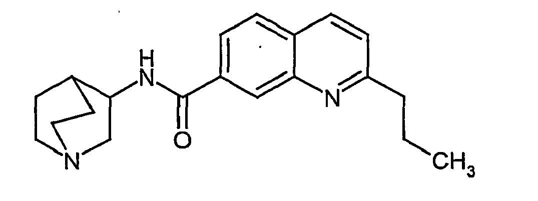

In Analogie zur Vorschrift für Beispiel 5 wurde Beispiel 4A mit 1-Hexen-3-ol umgesetzt. Man erhielt die Titelverbindung in 12 % Ausbeute.

LC/MS (Bedingungen wie bei Beispiel 6): Rt = 1,73 min; MS (ESI+): m/z = 324 [M+H]⁺.
¹H-NMR (400 MHz in D₆-DMSO) δ = 0,96 (t, 3H), 1,38 (m, 1H), 1,63 (m, 2H), 1,87 (m, 5H), 2,75 (m, 5H), 2,93 (m, 3H), 3,20 (m, 1H), 4,05 (m, 1H), 7,50 (d, 1H), 7,96 (m, 2H), 8,30 (m, 1H), 8,54 (s, 1H), 8,61 (d, 1H).

### Beispiel 13

### N-(1-Azabicyclo[2.2.2]oct-3-yl)-2-ethyl-4-methyl-7-chinolincarboxamid

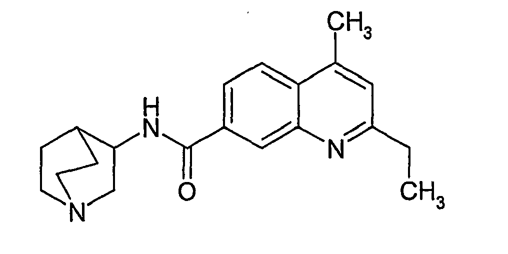

In Analogie zur Vorschrift für Beispiel 5 wurde Beispiel 4A mit 2-Hexin-4-ol umgesetzt. Man erhielt die Titelverbindung in 15 % Ausbeute.

LC/MS (Bedingungen wie bei Beispiel 6): Rt = 1,73 min; MS (ESI+): m/z = 324 [M+H]⁺.
¹H-NMR (400 MHz in D₆-DMSO) δ = 1,32 (t, 3H), 1,60 (m, 2H), 1,85 (m, 1H), 2,70 (m, 8H), 2,92 (m, 4H), 3,12 (m, 1H), 4,02 (m, 1H), 7,40 (s, 1H), 7,97 (d, 1H), 8,10 (d, 1H), 8,53 (m, 1H), 8,59 (d, 1H).

### Beispiel 14

### N-(1-Azabicyclo[2.2.2]oct-3-yl)-4-(tetrahydro-2H-pyran-2-yl)-6-chinolincarboxamid

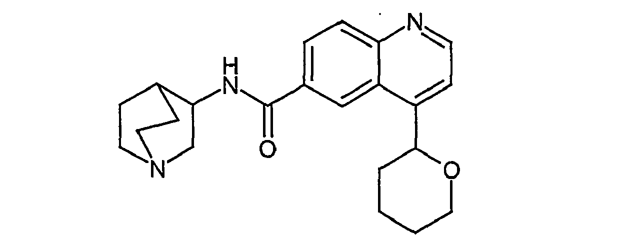

In Analogie zur Vorschrift für Beispiel 5 wurde Beispiel 2A mit 3-(Tetrahydro-2*H-*pyran-2-yl)-2-propin-1-ol umgesetzt. Man erhielt die Titelverbindung in 12 % Ausbeute.

LC/MS (Bedingungen wie bei Beispiel 6): Rt = 1,78 min; MS (ESI+): m/z = 366 [M+H]⁺.
¹H-NMR (400 MHz in D₆-DMSO) δ = 1,22- 2,05 (m, 13H), 2,71 (m, 5H), 2,90 (m, 2H), 3,17 (m, 1H), 3,76 (m, 1H), 4,00 (m, 1H), 4,16 (m, 1H), 5,18 (d, 1H), 7,60 (d, 1H), 8,05 (d, 1H), 8,18 (d, 1H), 8,52 (s, 1H), 8,94 (d, 1H).

### Beispiel 15

### N-(1-Azabicyclo[2.2.2]oct-3-yl)-4-(tetrahydro-2H-pyran-2-yl)-7-chinolincarboxamid

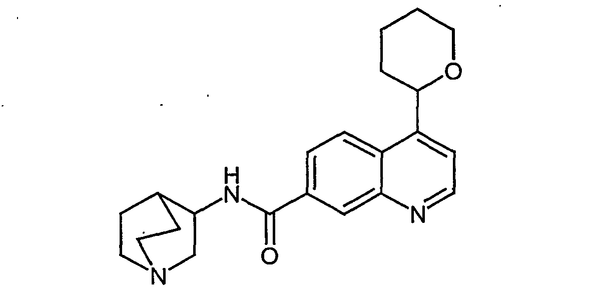

In Analogie zur Vorschrift für Beispiel 5 wurde Beispiel 4A mit 3-(Tetrahydro-2*H-*pyran-2-yl)-2-propin-1-ol umgesetzt. Man erhielt die Titelverbindung in 9 % Ausbeute.

LC/MS (Bedingungen wie bei Beispiel 6): Rt = 1,92 min; MS (ESI+): m/z = 366 [M+H]⁺.

### Beispiel 16

### N-(1-Azabicyclo[2.2.2]oct-3-yl)-2-phenyl-6-chinolincarboxamid

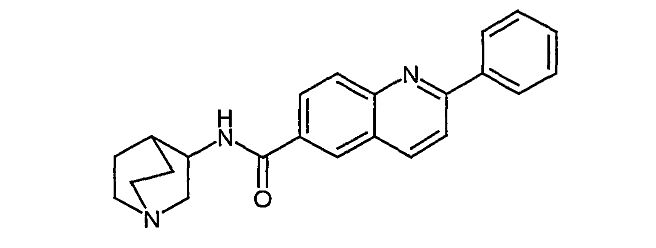

Gemäß der allgemeinen Vorschrift wurden Zwischenstufe 2 mit 3-Phenyl-1-propen-3-ol umgesetzt. Man erhielt die Titelverbindung in 38 % Ausbeute.

LC/MS (Bedingungen wie bei Beispiel 6): Rt = 1,94 min; MS (ESI+): m/z = 358 [M+H]⁺.

### Beispiel 17

### N-(1-Azabicyclo[2.2.2]oct-3-yl)-2-phenyl-7-chinolincarboxamid

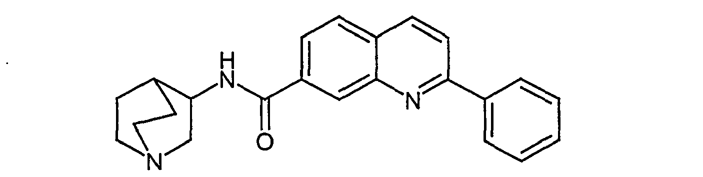

In Analogie zur Vorschrift für Beispiel 5 wurde Beispiel 4A mit 3-Phenyl-1-propen-3-ol umgesetzt. Man erhielt die Titelverbindung in 24 % Ausbeute.

LC/MS (Bedingungen wie bei Beispiel 6): Rt = 1,95 min; MS (ESI+): m/z = 358 [M+H]⁺.

## Patentansprüche

1. Verbindungen der allgemeinen Formel (I), 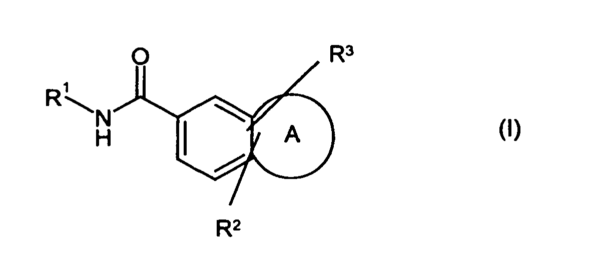 in welcher
R¹ für I-Aza-bicyclo[2.2.2]oct-3-yl steht
der Ring A für Pyrimido steht, oder für gegebenenfalls Benzo-kondensiertes Pyrido, Pyrazino oder Pyridazino steht,
und
R² und R³ gleich oder verschieden sind und für Reste ausgewählt aus der Gruppe Wasserstoff, Halogen, Formyl, Carbamoyl, Cyano, Trifluormethyl, Trifluormethoxy, Nitro, (C₁-C₆)-Alkyl, (C₁-C₆)-Alkoxy, (C₁-C₆)-Alkylthio, (C₃-C₈)-Cycloalkyl, 4- bis 8-gliedriges Heterocyclyl, Phenyl oder 5- bis 6-gliedriges Heteroraryl stehen, wobei Phenyl und Heteroaryl gegebenenfalls durch Halogen, (C₁-C₄)-Alkyl, (C₁-C₆)-Alkoxy, Amino, Mono- oder Di-(C₁-C₄)-Alkylamino, (C₁-C₄)-Alkanoylamino oder (C₁-C₄)-Alkansulfonylamino substituiert sind,
und deren Salze, Hydrate und/oder Solvate.

2. Verbindungen nach Anspruch 1, wobei
R¹ die in Anspruch 1 angegebene Bedeutung hat,
der Ring A zusammen mit dem ankondensierten Benzolrest für Chinolin-5-yl oder Chinolin-6-yl steht,
und
R² und R³ gleich oder verschieden sind und für Reste ausgewählt aus der Gruppe Wasserstoff, Halogen, Cyan, Trifluormethyl, Methyl und Ethyl stehen,
und deren Salze, Hydrate und/oder Solvate.

3. Verfahren zur Herstellung von Verbindungen der allgemeinen Formel (I) nach Anspruch 1, **dadurch gekennzeichnet, dass** man
Verbindungen der allgemeinen Formel (II),
R¹-NH₂ (II)
in welcher R¹ die oben genannte Bedeutung hat,
mit einer Verbindung der allgemeinen Formel (III), 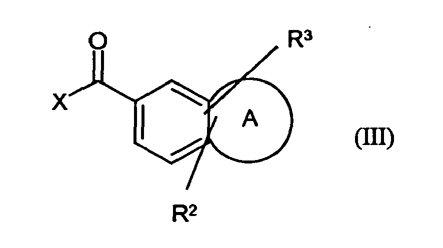 in welcher
der Ring A, R² und R³ die oben genannte Bedeutung haben, und
X für Hydroxy oder eine geeignete Abgangsgruppe steht,
in einem inerten Lösungsmittel gegebenenfalls in Gegenwart eines Kondensationsmittels und gegebenenfalls in Gegenwart einer Base umsetzt.

4. Verwendung von Verbindungen nach einem der Ansprüche 1 bis 3 zur Behandlung und/oder Prophylaxe von Krankheiten.

5. Arzneimittel enthaltend mindestens eine der Verbindungen nach einem der Ansprüche 1 bis 3 in Zusammenmischung mit mindestens einem pharmazeutisch verträglichen, im wesentlichen nichtgiftigen Träger oder Exzipienten.

6. Verwendung von Verbindungen nach einem der Ansprüche 1 bis 3 zur Herstellung eines Arzneimittels zur Verbesserung der Wahrnehmung, Konzentrationsleistung, Lernleistung und/oder Gedächtnisleistung.

7. Verwendung von Verbindungen nach einem der Ansprüche 1 bis 3 zur Herstellung eines Arzneimittels zur Behandlung und/oder Prophylaxe von Störungen der Wahrnehmung, Konzentrationsleistung, Lernleistung und/oder Gedächtnisleistung.

8. Verwendung eines Arzneimittels nach Anspruch 6 zur Behandlung und/oder Prophylaxe von Störungen der Wahrnehmung, Konzentrationsleistung, Lernleistung und/oder Gedächtnisleistung.

## Claims

1. Compounds of the formula (I) 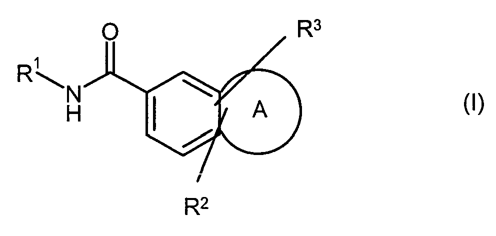 in which
R¹ is 1-azabicyclo[2.2.2]oct-3-yl,
the ring A is pyrimido, or is optionally benzo-fused pyrido, pyrazino or pyridazino,
and
R² and R³ are identical or different and are radicals selected from the group of hydrogen, halogen, formyl, carbamoyl, cyano, trifluoromethyl, trifluoromethoxy, nitro, (C₁-C₆)-alkyl, (C₁-C₆)-alkoxy, (C₁-C₆)alkylthio, (C₃-C₈)-cycloalkyl, 4- to 8-membered heterocyclyl, phenyl or 5- to 6-membered heteroaryl, where phenyl and heteroaryl are optionally substituted by halogen, (C₁-C₄)-alkyl, (C₁-C₄)-alkoxy, amino, mono- or di-(C₁-C₄)-alkylamino, (C₁-C₄)-alkanoylamino or (C₁-C₄)-alkanesulfonylamino.
and the salts, hydrates and/or solvates thereof.

2. Compounds according to Claim 1, where
R¹ has the meaning indicated in Claim 1,
the ring A together with the fused-on benzene residue is quinolin-5-yl or quinolin-6-yl,
and
R² and R³ are identical or different and are radicals selected from the group of hydrogen, halogen, cyano, trifluoromethyl, methyl and ethyl,
and the salts, hydrates and/or solvates thereof.

3. Process for preparing compounds of the formula (I) according to Claim 1, **characterized in that**
compounds of the general formula (II)
R¹-NH₂ (II)
in which R¹ has the abovementioned meaning,
are reacted with a compound of the general formula (III) 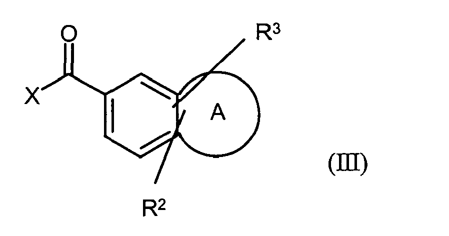 in which
X is hydroxyl or a suitable leaving group,
the ring A, R² and R³ have the abovementioned meaning, and in an inert solvent, where appropriate in the presence of a condensing agent, and where appropriate in the presence of a base.

4. Use of compounds according to any of Claims 1 to 3 for the treatment and/or prophylaxis of diseases.

5. Medicament comprising at least one of the compounds according to any of Claims 1 to 3 in combination with at least one pharmaceutically acceptable, essentially nontoxic carrier or excipient.

6. Use of compounds according to any one of Claims 1 to 3 for producing a medicament for improving perception, concentration, learning and/or memory.

7. Use of compounds according to any of Claims 1 to 3 for producing a medicament for the treatment and/or prophylaxis of impairments of perception, concentration, learning and/or memory.

8. Use of a medicament according to Claim 6 for the treatment and/or prophylaxis of impairments of perception, concentration, learning and/or memory.

## Revendications

1. Composés de formule générale (I) : 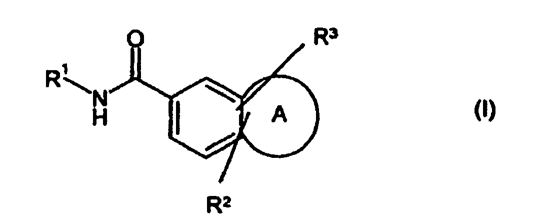 dans laquelle
R¹ représente un groupe 1-aza-bicyclo[2.2.2]oct-3-yle,
le noyau A représente un noyau pyrimido, ou représente un noyau pyrido, pyrazino ou pyridazino éventuellement condensé au benzène,
et
R² et R³ sont identiques ou différents et représentent des restes choisis dans le groupe hydrogène, halogéno, formyle, carbamoyle, cyano, trifluorométhyle, trifluorométhoxy, nitro, alkyle en C₁ à C₆, alkoxy en C₁ à C₆, alkylthio en C₁ à C₆, cycloalkyle en C₃ à C₈, hétérocyclyle tétragonal à octogonal, phényle ou hétéroaryle pentagonal ou hexagonal, les restes phényle et hétéroaryle étant éventuellement substitués par un radical halogéno, alkyle en C₁ à C₄, alkoxy en C₁ à C₆, amino, mono- ou di (alkyle en C₁ à C₉) amino, alcanoylamino en C₁ à C₄ ou alcanesulfonylamino en C₁ à C₄,
et leurs sels, leurs hydrates et/ou leurs produits de solvatation.

2. Composés suivant la revendication 1, dans lesquels
R¹ a la définition indiquée dans la revendication 1,
le noyau A forme conjointement avec le reste benzène condensé un noyau quinoléine-5-yle ou quinoléine-6-yle,
et
R² et R³ sont identiques ou différents et représentent des restes choisis dans le groupe hydrogène, halogéno, cyano, trifluorométhyle, méthyle et éthyle,
et leurs sels, leurs hydrates et/ou leurs produits de solvatation.

3. Procédé de production de composés de formule générale (I) suivant la revendication 1, **caractérisé en ce que** :
on fait réagir des composés de formule générale (II) :
**R¹-NH₂** **(II)**
dans laquelle R¹ a la définition indiquée ci-dessus,
avec un composé de formule générale (III) : 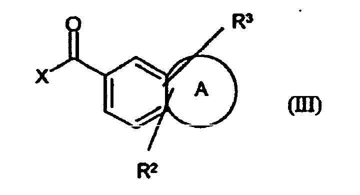
dans laquelle
le noyau A, R² et R³ ont la définition indiquée ci-dessus, et
X représente un groupe hydroxy ou un groupe partant convenable,
dans un solvant inerte, éventuellement en présence d'un agent de condensation et, le cas échéant, en présence d'une base.

4. Utilisation de composés suivant l'une des revendications 1 à 3, pour le traitement et/ou la prophylaxie de maladies.

5. Médicament contenant au moins l'un des composés selon l'une des revendications 1 à 3, en mélange avec au moins un support ou excipient pharmaceutiquement acceptable, essentiellement non toxique.

6. Utilisation de composés suivant l'une des revendications 1 à 3, pour la préparation d'un médicament destiné à améliorer la perception, la capacité de concentration, la capacité d'apprentissage et/ou la capacité de mémoire.

7. Utilisation de composés suivant l'une des revendications 1 à 3, pour la préparation d'un médicament destiné au traitement et/ou à la prophylaxie de troubles de la perception, de la capacité de concentration, de la capacité d'apprentissage et/ou de la capacité de mémoire.

8. Utilisation d'un médicament selon la revendication 6, pour le traitement et/ou la prophylaxie de troubles de la perception, de la capacité de concentration, de la capacité d'apprentissage et/ou de la capacité de mémoire.
